Europäisches Patentamt

European Patent Office

Office européen des brevets

(11) Veröffentlichungsnummer: **0 373 237 A1**

## (12) EUROPÄISCHE PATENTANMELDUNG

(21) Anmeldenummer: 88120823.5

(22) Anmeldetag: 13.12.88

(51) Int. Cl.⁵: **A61M 15/00, B05B 17/06**

(43) Veröffentlichungstag der Anmeldung:
**20.06.90 Patentblatt 90/25**

(84) Benannte Vertragsstaaten:
**AT BE CH DE ES FR GB GR IT LI NL SE**

(71) Anmelder: **Siemens Aktiengesellschaft**
**Wittelsbacherplatz 2**
**D-8000 München 2(DE)**

Anmelder: **BOEHRINGER INGELHEIM KG**
**Binger Strasse 173**
**D-6507 Ingelheim am Rhein(DE)**

(72) Erfinder: **van der Linden, Klaus, Dipl.-Ing.**
**Innerer Ring 40**
**D-8640 Kronach(DE)**
Erfinder: **Friedrich, Jürgen**
**Forststrasse 21**
**D-8621 Weidhausen(DE)**
Erfinder: **Zierenberg, Berg, Dr. rer nat.**
**Goethestrasse 1**
**D-6530 Bingen(DE)**

(74) Vertreter: **Fuchs, Franz-Josef, Dr.-Ing. et al**
**Postfach 22 13 17**
**D-8000 München 22(DE)**

(54) **Inhalationsgerät, insbesondere Taschen-Inhalationsgerät.**

(57) Das Inhalationsgerät besitzt ein Gehäuse (2), einen im Gehäuse (2) angeordneten Ultraschall-Zerstäuber (8), eine Einrichtung (40) zur Zuführung eines Medikaments zum Zerstäuber (8) und ein Mundstück (30), das an den Aerosol-Raum (26) vor dem Mundstück (30) anschließt. Um einen Niederschlag des zerstäubten Medikaments (Aerosol) an den Wänden des Aerosol-Raums (26) zu verhindern, ist nach der Erfindung für eine Durchströmung dieses Raums (26) gesorgt. Dafür ist eine im Gehäuse (2) vorgesehene Lufteinlaß-Öffnung (28) vorgesehen, die mit dem Aerosol-Raum (26M ]1 in Verbindung steht. Weiterhin sind Laminarisierungsmittel (22) für die angesaugte Luft vorgesehen.

FIG 1

## Inhalationsgerät, insbesondere Taschen-Inhalationsgerät

Die Erfindung bezieht sich auf ein Inhalationsgerät, insbesondere Taschen-Inhalationsgerät, mit

a) einem Gehäuse,

b) einem im Gehäuse angeordneten Ultraschall-Zerstäuber,

c) einer Einrichtung zur Zuführung eines Medikaments zum Ultraschall-Zerstäuber und

d) einem Mundstück, das an den Raum vor dem Ultraschall-Zerstäuber anschließt.

Ein solches Taschen-Inhalationsgerät ist beispielsweise aus der europäischen Patentanmeldung 0 258 637 bekannt. Das bekannte Gerät ist zum Zerstäuben von Arzneimitteln für Inhalationszwecke vorgesehen. Es ist so konzipiert, daß es ein Aerosol mit Tröpfchen erzeugt, deren Durchmesser im wesentlichen im Bereich von 1 bis 5 µm liegt. Somit ist es bevorzugt für Asthmatiker geeignet. Das bevorzugt aus Kunststoff bestehende Gehäuse umfaßt ein Unterteil, in dem ein piezoelektrisches Schwingsystem mit Zerstäuberteller, eine Elektronikschaltung zur Ultraschall-Anregung dieses Systems, ein Akkumulator zur Energieversorgung der Elektronikschaltung sowie ein magnetisch betätigbarer Schalter zum Einschalten der Ultraschall-Anregung untergebracht sind. Das Gehäuse umfaßt weiter ein auf dem Unterteil lösbar befestigtes Oberteil mit einem Mundstück oder Ansaugstutzen, in dessen Richtung das Medikament zerstäubt wird, und mit einem Aufnahmeraum, in den eine Medikamenten-Dosierkartusche eingesetzt werden kann. Diese Dosierkartusche ist mittels eines aufgesetzten Drückers oder Knopfes gegen eine eingebaute Feder in sich verschiebbar. Beim Betätigen des Drückers wird die Kartusche in Richtung auf den Zerstäuberteller verschoben; gleichzeitig wird sie in sich verschoben. Dabei wird ein Tröpfchen von der Dosieröffnung der Kartusche am Zerstäuberteller abgestreift. Ein an der Kartusche angeordneter Magnet betätigt beim Drücken magnetisch den Schalter, worauf nach einer vorgegebenen Zeitspanne der Ultraschall-Zerstäuber mit Zerstäuberteller angeregt wird, woraufhin das abgestreifte Tröpfchen vernebelt wird. Das entstandene Aerosol kann nun über das Mundstück vom Benutzer eingeatmet werden. Das Gerät kann klein, leicht und handlich ausgeführt werden; der Benutzer kann es daher ohne Schwierigkeiten bei jeder Gelegenheit mit sich führen. Die Auffüllung mit dem zu zerstäubenden Medikament erfolgt durch einfaches Auswechseln der Dosierkartusche.

Eine Dosierkartusche der hier betrachteten Art ist beispielsweise aus der DE-OS 33 39 180 bekannt. Diese besitzt ein zylindrisches Frontteil, das in seiner Stirnwand zentral ein durchgehendes Dosierröhrchen mit außen gelegener Dosieröffnung trägt und in seinem Innenraum eine Spiralfeder enthält. In dieses Frontteil ragt rückseitig ein dünneres, das Medikament enthaltendes zylindrisches Rückteil mit zwei Stirnwänden. Beim Drücken kann das Rückteil in das Frontteil gleiten. Ein Austrittsröhrchen, das an der im Innenraum des Vorderteils gelegenen Stirnwand angebracht ist, sitzt gleitend auf dem Rückteil des Dosierröhrchens. Die Dosierkartusche ist somit in sich verschiebbar. Wird das Rückteil gegen die Kraft der Feder in den Innenraum des Frontteils gedrückt, so wird ein kleiner Tropfen des Medikaments über die Dosieröffnung des Dosierröhrchens abgegeben.

Es hat sich gezeigt, daß sich bei dem bekannten Taschen-Inhalationsgerät (EP-A-0 258 637) beim Zerstäuben ein Teil des Aerosols an den Wänden im Aerosol-Raum vor dem Ultraschall-Zerstäuber und/oder im Mundstück niederschlägt und damit für die medizinische Behandlung verlorengeht. Dieser Niederschlag ist aus verschiedenen Gründen unerwünscht; er kann nur schwer entfernt werden, wobei die Gefahr der Beschädigung des Zerstäubers und des Zerstäubertellers besteht.

Der Erfindung liegt die Aufgabe zugrunde, ein Inhalationsgerät der eingangs genannten Art so auszugestalten, daß die Gefahr für die Bildung eines Niederschlags des Aerosols deutlich verringert wird.

Diese Aufgabe wird erfindungsgemäß gelöst durch eine im Gehäuse angebrachte Lufteinlaß-Öffnung, die mit dem Raum vor dem Ultraschall-Zerstäuber in Verbindung steht.

Über diese Lufteinlaß-Öffnung kann der Benutzer Luft in den Aerosol-Raum saugen; diese Luft reißt das dort befindliche Aerosol mit, so daß die Wahrscheinlichkeit eines Niederschlags deutlich reduziert ist.

Eine besonders vorteilhafte Weiterbildung sieht vor, daß die Lufteinlaß-Öffnung mit dem besagten Raum über ein Mittel zur Laminarisierung der angesaugten Luft in Verbindung steht. Dabei ist bevorzugt das Mittel zur Laminarisierung eine von kleinen Löchern durchsetzte Wand. Von der laminaren Luftströmung wird das Aerosol mitgerissen, so daß eine besonders geringe Wahrscheinlichkeit des Aerosol-Niederschlags an den Wänden vor dem Austritt aus dem Gerät besteht.

Weitere vorteilhafte Ausgestaltungen der Erfindung sind in den Unteransprüchen gekennzeichnet.

Ein Ausführungsbeispiel der Erfindung wird im folgenden anhand von vier Figuren näher erläutert. Es zeigen:

FIG 1 ein Taschen-Inhalationsgerät mit eingesetzter Dosierkartusche im Längsschnitt;

FIG 2 das Gerät von Figur 1 im Querschnitt entlang der Linie II-II;

FIG 3 das Gerät von Figur 1 im Querschnitt entlang der Linie III-III; und

FIG 4 das Gerät von Figur 1 im Schnitt entlang der Linie IV-IV, wobei die Dosierkartusche und die Lasche nicht gezeigt und die Fenster herausgenommen sind.

Das dargestellte Taschen-Inhalationsgerät umfaßt ein Gehäuse 2 aus Kunststoff mit einem Unterteil 2a und einem darauf lösbar befestigten Oberteil 2b. Die lösbare Befestigung ist durch zumindest eine federnde Seitenwand 4 und einen Schnapp- oder Rastverschluß 6 sichergestellt.

Im Unterteil 2a ist ein piezoelektrisches Schwingsystem oder Ultraschall-Zerstäuber 8 mit nach oben weisendem Zerstäuberteller 10 untergebracht. Dieser Zerstäuber 8 arbeitet mit einer Betriebsfrequenz im Bereich von 1 bis 5 MHz. Die elektrische Energie hierfür wird von einer Elektronikschaltung 12 bereitgestellt, die sich in einer abgeschlossenen vorderen Kammer befindet. In dieser Kammer ist auch ein magnetisch betätigbarer Schalter 14, insbesondere ein Reed-Kontakt, untergebracht. Er befindet sich an der oberen Wand 16 des Unterteils 2a, die auch als Zwischenwand bezeichnet werden kann. In einer hinteren Kammer ist ein Akkumulator 18 austauschbar eingesetzt. Er versorgt die Elektronikschaltung 12 mit elektrischer Energie. Die vordere und die hintere Kammer sind flüssigkeitsdicht abgeschlossen. Zu diesem Zweck ist der Ultraschall-Zerstäuber 8 in der vorderen Kammer bevorzugt zum Beispiel mit einer Vergußmasse 20 eingegossen.

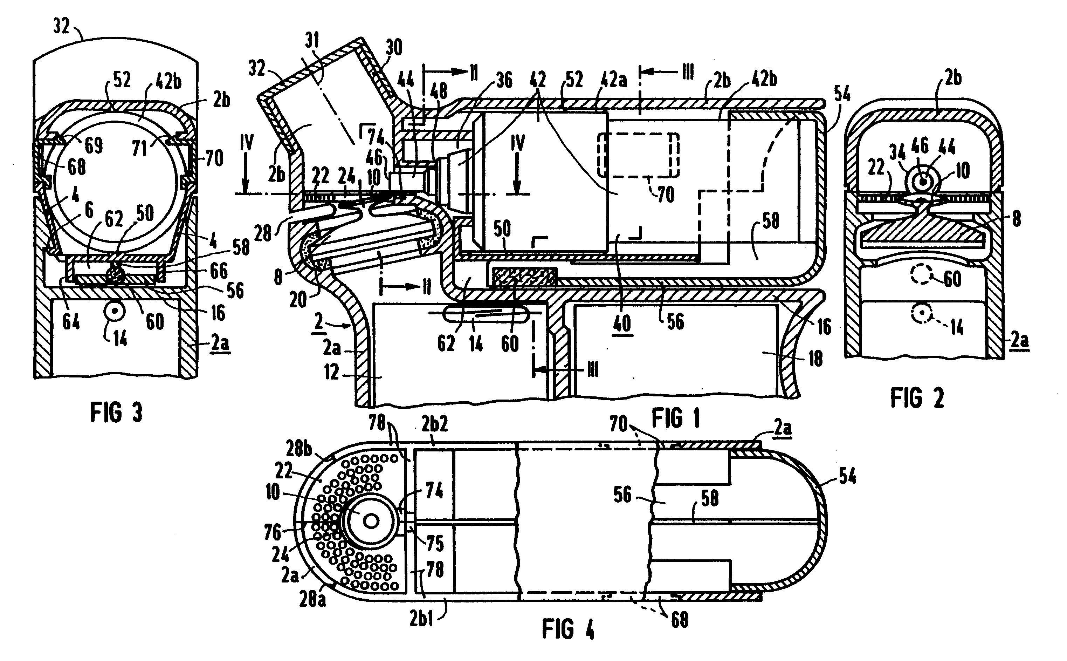

Im Bereich des Ultraschall-Zerstäubers 8, und zwar in seiner unmittelbaren Nähe, ist eine mit kleinen runden Löchern versehene Siebplatte oder Abschlußwand 22 vorgesehen. Stattdessen könnte sie auch mit Schlitzen oder rechteckigen Durchbrüchen versehen sein. Die Wand 22 ist etwa halbringförmig geformt. Sie besitzt im zentralen Teil eine größere Öffnung 24, in die der Zerstäuberteller 10 mit seiner Zerstäuberfläche hineinragt. Zwischen dem Zerstäuberteller 10 und dem Rand der Öffnung 24 ergibt sich somit ein etwa ringförmiger Trennspalt. Die Abschluß wand 22 hat zwei Funktionen: Zum einen dient sie zum mechanischen Schutz des Zerstäubers 8 und des Zerstäubertellers 10 vor Beschädigungen, und zum anderen wird infolge der Vielzahl kleiner Öffnungen im darüberliegenden Raum 26, der als Aerosol-Raum bezeichnet werden kann, eine laminare Strömung erreicht. Diese tritt auf, sobald Luft über eine Lufteinlaßöffnung 28, die mit dem Aerosol-Raum 26 in Verbindung steht und die bevorzugt als Schlitz mit Schlitzrändern 28a und 28b (vergl. Fig. 4) ausgebildet ist, angesogen wird. Die Wand 22 kann somit als Mittel zur Laminarisierung der angesaugten Luft angesehen werden. Auch andere solche Mittel können vorliegend eingesetzt werden. Die Lufteinlaßöffnung 28 ist vorliegend unmittelbar unterhalb der Wand 22 im Gehäuse 2 angeordnet.

Die Oberfläche des Zerstäubertellers 10 steht leicht schräg zur Wand 22, die auch als Lochgitter bezeichnet werden kann. Diese Schrägstellung des Ultraschall-Zerstäubers 8 mit Richtung auf den Mund des Benutzers trägt zu einem kompakten Aufbau des Inhalationsgeräts bei. Zugleich sorgt sie für eine Zerstäubung in Richtung der Austrittsöffnung des Aerosol-Raums 26. Durch die Lochung der Wand 22 ist eine ausreichende Reinigung des Schwingerbereiches möglich.

Der Aerosol-Raum 26 ist im vorderen Teil des Oberteils 2b angeordnet. Er läuft in ein im wesentlichen ovales Mundstück 30 aus, dessen Ausgangsöffnung mit einem Kunststoff-Deckel 32 verschließbar ist. Dabei kann die Ebene der Wand 22 im Winkel von etwa 45$^\circ$ zur Achse 31 des Mundstücks 30 ausgerichtet sein.

Der Aerosol-Raum 26 ist über eine Führungsöffnung 34 mit einem hinteren Raum 36 verbunden, in dem eine generell mit 40 bezeichnete Einrichtung zur Zuführung eines Medikaments zum Ultraschall-Zerstäuber 10 untergebracht ist. Diese Einrichtung 40 umfaßt eine an sich bekannte, in sich verschiebbare Dosierkartusche 42 mit einem zylindrischen Vorderteil 42a und einem darin verschiebbaren zylindrischen Rückteil 42b, das das Medi kament enthält. Am Vorderteil 42a ist ein Dosierröhrchen 44 mit hinteren Absätzen als Kartuschenspitze angebracht. Dessen Dosieröffnung ist mit 46 bezeichnet. Das Dosierröhrchen 44 liegt bei liegend eingeschobener Dosierkartusche 42 unter Freilassung eines Ringspalts in der Führungsöffnung 34. Diese Führungsöffnung 34 dient also der exakten Positionierung. Wichtig ist dabei, daß die Spitze der Dosierkartusche 42 mit einem der genannten Absätze an einem Anschlag 48 anliegt, der z. B. durch die Wand der Dosieröffnung 46 gebildet wird. Dieser Anschlag 48 ist wichtig für die Zentrierung, aber auch für das Festhalten der Dosierkartusche 42 beim Dosieren. Die Zentrierung und Führung wird im übrigen auch durch Längsrippen 50, 52 im Raum 36 sichergestellt.

Die Einrichtung 40 zur Zuführung des Medikaments umfaßt weiter einen endseitig auf die Dosierkartusche 42 aufgesetzten Drücker 54. Der Drücker 54 ist dabei auf das Ende der Dosierkartusche 42 fest aufgeschoben; er ist aber - beim Austauschen der Dosierkartusche 42 - auch ohne weiteres von diesem Ende wieder abziehbar. Die Dimensionierung ist so getroffen, daß der Drücker 54 in der ungedrückten Normalstellung mit seiner Rückfläche etwa das Gehäuse 2 hinten abschließt; bei Betätigung wird er weiter in das Oberteil 2b eingeschoben. Der Drücker 54 selbst dient damit zur exakten Halterung der Kartusche 42.

Aus einem Vergleich von Figur 1 und Figur 4 ergibt sich, daß der Drücker 54 als halbzylindrischer Hohlkörper ausgebildet ist. Seine untere Stirnwand oder untere Seite läuft in eine Lasche 56 aus. Mit anderen Worten, die Lasche 56 ist an den Drücker 54 angearbeitet. Diese Lasche 56 ist dabei im wesentlichen ein rechteckförmiges Kunststoffteil. Sie ist an ihrer Oberseite bevorzugt mit einer Längsrippe 58 versehen, um die Stabilität zu erhöhen. Wie später deutlich werden wird, dient die Oberkante dieser Längsrippe 58 ebenso wie die Unterseite der Lasche 56 als Gleitfläche, und zwar sowohl beim Einsetzen der Kartusche 42 als auch beim Dosieren.

Am vorderen Ende der Lasche 56 ist ein Magnet 60 befestigt. Bei aufgeschobenem Drücker 54 liegen die Lasche 56, die Längsrippe 58 und der Magnet 60 in einem Raum 62 zwischen dem Unterteil 2a und dem Oberteil 2b. In diesem Raum 62 ist die Lasche 56 bei Betätigung des Drückers 54 längsverschiebbar. Verschmutzungen sind nicht zu befürchten. Beim Verschieben wird der Magnet 60 relativ zum Schalter 14 nach links verschoben. Gelangt er in dessen magnetischen Einflußbereich, so wird der Schalter 14 betätigt. Hierdurch wird - nach Loslassen des Drückers 54 und beim Öffnen des Schalters 14 - mit Hilfe der Elektronikschaltung 12 eine Ultraschall-Erregung des Zerstäubers 8 ausgelöst. Die Lasche 56, die Längsrippe 58 und der Drücker 54 können als gemeinsames Spritzgußteil ausgebildet sein. Aus Figur 1 wird deutlich, daß der Magnet 60, der z. B. zylinderförmig geformt sein kann, in die Lasche 56 und die Längsrippe 58 eingelassen ist, und daß er im wesentlichen bündig mit der dem Schalter 14 zugewandten Fläche der Lasche 56 abschließt. Bei Betätigung wird der Magnet 60 entlang der Wand 16 des Gehäuses 2 verschoben. Bei Entlastung durch den Finger des Benutzers fährt die Kartusche 42 infolge ihrer Federkraft (und damit auch der Magnet 60) wieder in ihre dargestellte Ausgangsstellung zurück.

Die Führung der Lasche 56 im Raum 62 erfolgt mit Hilfe von zwei Seitenwänden 64, 66, die in einigem Abstand parallel zueinander an der Unterseite des Oberteils 2b befestigt sind.

An den beiden parallelen Seitenlängswänden 2b1, 2b2 des Oberteils 2b sind einander gegenüberliegend zwei Fenster 68, 70 angeordnet, über die eine Beobachtung des Füllstands der durchsichtigen, aus Kunststoff bestehenden Kartusche 42 möglich ist. Die Dosierkartusche 42 liegt dabei im eingeschobenen Zustand zwischen diesen beiden Fenstern 68, 70. Die Fenster 68, 70 sind bevorzugt aus Kunststoff ausgeführt und mit Rastnasen 69, 71 ausgestattet, so daß sie in entsprechende Öffnungen in den Seitenlängswänden des Oberteils 2b eingeklipst werden können. Diese Rastnasen 69, 71 sind so geformt, daß sie eine Sicherung gegen ein unbeabsichtigtes Herausziehen oder Herausfallen der Dosierkartusche 42 bilden.

Im Bereich des Zerstäubertellers 10 ist an der dort vorhandenen Führungsöffnung 34 eine Senke oder Eindellung 74 im Material vorgesehen. Dadurch wird die Kapillarwirkung im Ringspalt der Führungsöffnung 34 für das austretende Medikamententröpfchen beim Dosieren herabgesetzt. Mit anderen Worten: Der Abstand zwischen der Austrittsöffnung 46 und der Oberfläche des Zerstäubertellers 10 ist für das austretende Dosiertröpfchen kleiner als zwischen der Dosieröffnung 46 und dem Boden der Senke 74. Hierdurch wird die Körperanhangskraft des Zerstäubertellers 10 bezüglich des Tröpfchens unterstützt, so daß beim Dosieren gewährleistet ist, daß dieses auf der Oberfläche des Zerstäubertellers 10 plaziert wird und nicht etwa im Bereich des Ringspalts an der Öffnung 24. Ein eventuelles Rückfließen des Medikamententröpfchens über eine mögliche Kapillare zwischen dem Außendurchmesser der Kartuschenspitze und der Führungsöffnung des Oberteils 2b wird also durch einen entsprechend vergrößerten Führungsöffnungsdurchmesser erreicht.

Das Unter- und Oberteil 2a bzw. 2b kann jeweils aus zwei Hälften oder Schalen zusammengesetzt sein. Dies ist in Figur 4 für das Unterteil 2a durch die Ansatzlinie 76 veranschaulicht.

Wie bereits früher dargelegt, ist das Oberteil 2b von oben auf das Unterteil 2a aufsetzbar und dort mit Hilfe des Rastverschlusses 6 einrast- oder einklemmbar. Drückt der Benutzer nun auf den Drücker 54, so wird das Rückteil 42b in das Vorderteil 42a der Dosierkartusche 42 eingedrückt und ein Medikamententröpfchen an den Zerstäuberteller 10 abgegeben. Gleichzeitig bewegt sich der an der Lasche 56 befestigte Magnet 60 mit dieser in Richtung auf den Schalter 14 und schließt denselben. Nach Loslassen des Drückers 54 und Öffnen des Schalters 14 wird aufgrund des Schaltvorgangs das Tröpfchen auf dem Zerstäuberteller 10 durch Ultraschallschwingungen zerstäubt. Es wird in ein Aerosol umgewandelt, das in den Raum 22 übergeht. Dieses kann vom Benutzer über das Mundstück 30 (bei abgenommenem Deckel 32) eingeatmet werden. Beim Einatmen wird Luft über die Lufteinlaßöffnung 28 eingesogen. Infolge der durch die Siebplatte 22 bewirkten laminaren Störung findet nur ein geringer Niederschlag des Medikaments an den Innenflächen des Raums 26 statt. Dies ist ein erheblicher Vorteil für die Betriebssicherheit und die Hygiene.

Es soll noch betont werden, daß das Laminarisierungsmittel prinzipiell auch im Oberteil 2b angeordnet sein kann, z. B. ebenfalls in Form eines Gitters.

**Ansprüche**

1. Inhalationsgerät, insbesondere Taschen-Inhalationsgerät, mit

a) einem Gehäuse (2),

b) einem im Gehäuse (2) angeordneten Ultraschall-Zerstäuber (8),

c) einer Einrichtung (40) zur Zuführung eines Medikaments zum Ultraschall-Zerstäuber (8) und

d) einem Mundstück (30), das an den Raum (26) vor dem Ultraschall-Zerstäuber (8) anschließt, **gekennzeichnet** durch eine im Gehäuse (2) angebrachte Lufteinlaß-Öffnung (28), die mit dem Raum (26) vor dem Ultraschall-Zerstäuber (8) in Verbindung steht.

2. Inhalationsgerät nach Anspruch 1, **dadurch gekennzeichnet,** daß die Lufteinlaß-Öffnung (28) mit dem besagten Raum (26) über ein Mittel zur Laminarisierung angesaugter Luft in Verbindung steht.

3. Inhalationsgerät nach Anspruch 2, **dadurch gekennzeichnet,** daß das Mittel zur Laminarisierung eine von kleinen Löchern durchsetzte Wand (22) ist.

4. Inhalationsgerät nach Anspruch 3, **dadurch gekennzeichnet,** daß die Wand (22) in unmittelbarer Nähe des Ultraschall-Zerstäubers (8) angeordnet ist.

5. Inhalationsgerät nach Anspruch 3 oder 4, **dadurch gekennzeichnet,** daß die Wand (22) eine größere Öffnung (24) aufweist, in die die Zerstäuberfläche (10) des Ultraschall-Zerstäubers (8) hineinragt.

6. Inhalationsgerät nach einem der Ansprüche 3 bis 5, **dadurch gekennzeichnet,** daß die Wand (22) bezüglich der Zerstäuberfläche (10) des Ultraschall-Zerstäubers (8) geneigt ist.

7. Inhalationsgerät nach Anspruch 6, **dadurch gekennzeichnet,** daß die Ebene der Wand (22) im Winkel von etwa 45° zur Achse (31) des Mundstücks (30) ausgerichtet ist.

8. Inhalationsgerät nach einem der Ansprüche 1 bis 7, **dadurch gekennzeichnet,** daß die Lufteinlaß-Öffnung (28) als Schlitz ausgebildet ist.

9. Inhalationsgerät nach einem der Ansprüche 3 bis 8, **dadurch gekennzeichnet,** daß die Lufteinlaß-Öffnung (28) in unmittelbarer Nähe der Wand (22) unterhalb derselben angeordnet ist.

10. Inhalationsgerät nach einem der Ansprüche 3 bis 9, **dadurch gekennzeichnet,** daß die Wand (22) etwa halbringförmig ausgebildet ist.

11. Inhalationsgerät nach einem der Ansprüche 1 bis 10, **dadurch gekennzeichnet,** daß das Gehäuse (2) ein Unterteil (2a) und ein darauf lösbar befestigbares Oberteil (2b) umfaßt, daß die Einrichtung (40) zur Zuführung des Medikaments und das Mundstück (30) im bzw. am Oberteil (26) angeordnet wird, und daß die Lufteinlaß-Öffnung (28), der Ultraschall-Zerstäuber (8) und das Mittel (22) zur Laminarisierung angesaugter Luft im Unterteil (2a) angeordnet sind.

12. Inhalationsgerät nach einem der Ansprüche 1 bis 11, **dadurch gekennzeichnet,** daß die Einrichtung (40) zur Zuführung des Medikaments als in sich verschiebbare Dosierkartusche (42) ausgebildet ist, und daß bevorzugt die Dosieröffnung (46) am Vorderteil (42a) der Dosierkartusche (42) beim Dosiervorgang bezüglich des Ultraschall-Zerstäubers (8) festgehalten ist, insbesondere durch einen Anschlag (48).

FIG 3

FIG 1

FIG 2

FIG 4

EP 0 373 237 A1

88 P 8 6 13 E

**Europäisches Patentamt**

# EUROPÄISCHER RECHERCHENBERICHT

Nummer der Anmeldung

EP 88 12 0823

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,X | EP-A-0 258 637 (SIEMENS AG) <br> * Figuren 1,2; Spalte 3, Zeilen 25-28; Spalte 3, Zeile 46 - Spalte 4, Zeile 7; Spalte 4, Zeilen 20-25 * | 1,8,11, 12 | A 61 M 15/00 <br> B 05 B 17/06 |
| Y | | 2 | |
| Y | FR-A-2 285 930 (SIEMENS AG) <br> * Figuren 1,2; Seite 2, Zeilen 17-26 * <br> --- | 2 | |
| X | BE-A- 531 640 (SOPROTEC) <br> * Figuren 1-3; Seite 2, Zeilen 3-26 * <br> --- | 1 | |
| X | DE-B-1 103 522 (VEB TRANSFORMATOREN- UND RÖNTGENWERK) <br> * Figur 1; Spalte 2, Zeile 48 - Spalte 3, Zeile 8 * <br> --- | 1 | |
| X | DE-A-2 524 862 (TDK) <br> * Figur 1; Seite 1, Zeilen 9-11; Seite 5, Zeilen 2-14 * <br> --- | 1 | |
| X | US-A-4 109 863 (OLSEN et al.) <br> * Figuren; Spalte 1, Zeilen 11-17; Spalte 3, Zeilen 8-52 * <br> --- | 1 | **RECHERCHIERTE SACHGEBIETE (Int. Cl.5)** <br><br> A 61 M <br> B 05 B |
| A | FR-A-2 444 504 (BOSCH-SIEMENS HAUSGERÄTE GmbH) <br> * Figuren 2,4; Seite 5, Zeile 28 - Seite 6, Zeile 24 * <br> ----- | 12 | |

Der vorliegende Recherchenbericht wurde für alle Patentansprüche erstellt

| Recherchenort | Abschlußdatum der Recherche | Prüfer |
|---|---|---|
| DEN HAAG | 03-07-1989 | JONES T.M. |